(19) Europäisches Patentamt
European Patent Office
Office européen des brevets

(11) Veröffentlichungsnummer: **0 303 733 B1**

(12) # EUROPÄISCHE PATENTSCHRIFT

(45) Veröffentlichungstag der Patentschrift: **16.12.92**

(51) Int. Cl.⁵: **C07D 495/04**, C07F 9/547, B01J 41/04, //(C07D495/04, 333:00,235:00)

(21) Anmeldenummer: **87113493.8**

(22) Anmeldetag: **15.09.87**

Die Akte enthält technische Angaben, die nach dem Eingang der Anmeldung eingereicht wurden und die nicht in dieser Patentschrift enthalten sind.

(54) **Verfahren zur Herstellung von Thiophenderivaten und die dabei erhaltenen Zwischenproduke.**

(30) Priorität: **17.08.87 DE 3727428**

(43) Veröffentlichungstag der Anmeldung:
**22.02.89 Patentblatt 89/08**

(45) Bekanntmachung des Hinweises auf die Patenterteilung:
**16.12.92 Patentblatt 92/51**

(84) Benannte Vertragsstaaten:
**DE FR GB IT**

(56) Entgegenhaltungen:
**EP-A- 0 069 881**
**WO-A-86/02069**
**DE-A- 3 018 109**
**DE-B- 2 417 992**

**JOURNAL OF THE AMERICAN CHEMICAL SOCIETY Band 70, 1948, Seiten 657-662; R. DUSCHINSKY et al.: "Synthesis of imidazolones structurally related to biotin by means of N-bromosuccinimide"**

(73) Patentinhaber: **Blum, Holger**
**Parkallee 75**
**W-2000 Hamburg 13(DE)**

(72) Erfinder: **Blum, Holger**
**Parkallee 75**
**W-2000 Hamburg 13(DE)**

(74) Vertreter: **Patentanwälte Kirschner & Grosse**
**Forstenrieder Allee 59**
**W-8000 München 71(DE)**

**EP 0 303 733 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

JOURNAL OF THE AMERICAN CHEMICAL SOCIETY Band 67, 1945, Seiten 2079-2084; R. DUSCHINSKY et al.: "A simple synthesis of d1-desthiobiotin and related substances"

CHEMICAL ABSTRACTS Band 81, Nr. 25, 23. Dezember 1974, Seite 542, Abstract Nr. 169 475s; T. TAGUCHI et al.: "Biotin synthesis. I. Synthesis of 4-(4-carboxy-butyl)-1,2-dihydrothieno/3,4-dimidazol-2-one"

CHEMICAL ABSTRACTS Band 69, Nr. 21, 18. November 1968, Seite 8124, Abstract Nr. 86 911g; I. ISAKA et al.: "Synthesis of biotin. I. Catalytic hydrogenation of 4,5-disubstituted 4-imidazolin-2-ones"

CHEMICAL ABSTRACTS Band 83, Nr. 25, 22. Dezember 1975, Seite 385, Abstract Nr. 206 165e; S.I. ZAV YALOV et al.: "Synthesis of 1-alkylderivatives of 2,3,4,5-tetradehydrobiotin"

CHEMICAL ABSTRACTS Band 94, Nr. 3, 19. Januar 1976, Seite 434, Abstract Nr. 15 640y; S.I. ZAV YALOV et al.: "Synthesis of 2,3,4,5-tetradehydrobiotin esters."

CHEMICAL ABSTRACTS Band 84, Nr. 15, 12. April 1976, Seite 569, Abstract Nr. 105 481v; S.I. ZAV YALOV et al.: "Synthesis of 2,3,4,5-tetradehydrobiotin esters."

CHEMIE-INGENIEUR-TECHNIK Band 51, Nr. 7, 1979, Seiten 728-736; F. MARTINOLA: "Ionenaustauscher und Adsorber-vielseitige Hilfsmittel der chemischen Industrie"

**Beschreibung**

Die Erfindung betrifft ein verbessertes Verfahren zur Herstellung von Thiophenderivaten der weiter unten angegebenen allgemeinen Formel (I) durch Umsetzung einer Verbindung der weiter unten angegebenen allgemeinen Formel (II) mit einem stark basischen quaternären Ammonium-Anionenaustauscherharz in der Monothiophosphatform sowie die bei dieser Synthese als Zwischenprodukt auftretende neue polymere Verbindung der weiter unten angegebenen allgemeinen Formel (III).

Thiophenderivate der Formel (I) können unter geeigneten Bedingungen durch Hydrierung in das Vitamin (±)-Biotin überführt werden. Geeignete Bedingungen für die katalytische Hydrierung sind in "Helvetica Chimica Acta", 59 (1976), Seite 1 005, sowie in DE-OS 30 18 109 beschrieben.

Es ist auch bereits bekannt, Verbindungen der Formel (II) mit Thioessigsäure, Thioharnstoff, Thioharnstoffacylverbindungen oder mit Natriumthiosulfat umzusetzen unter Bildung S-haltiger Zwischenprodukte der Formel

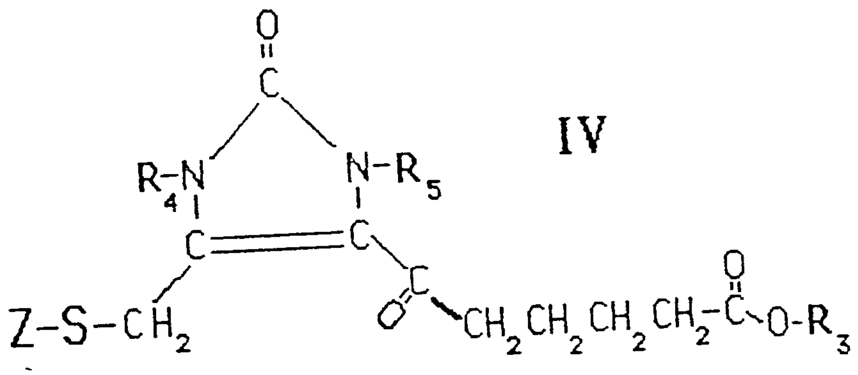

IV

worin $R_3$ niederes Alkyl, $R_4$ und $R_5$ niederes Acyl und Z einer der unten beschriebenen Reste ist.

Diese S-haltigen Zwischenprodukte der Formel (IV) können für $Z = CH_3CO$- (Thioacetat) nach einem von Taguchi et al in "Chemistry Letters" (1974), Seiten 729 bis 730, beschriebenen Verfahren hergestellt werden.

Zwischenprodukte der Formel (IV) für $Z = C(=NH)-NH_2 \cdot HBr$ (Thioharnstoff) können auch nach einem von Isaka et al in "Yakugaku Zasshi", 88 (4) (1968), Seiten 422 bis 427, beschriebenen Verfahren hergestellt werden. Zwischenprodukte der Formel (IV) mit $Z = C(=NH)-NHCOPh.HBr$ (Benzoylthioharnstoff) und mit $Z = -SO_2-O-Na$ (Thiosulfat) können nach einem Verfahren hergestellt werden, wie es von Zavyalov et al in "Izvestia Akademia Nauk. SSSR Ser. Chim. (1980), Seiten 1 943 bis 1 945, beschrieben ist. Nach Taguchi et al (vgl. "Chemistry Letters" (1974), Seiten 729 bis 730) wird das Zwischenprodukt der Formel (IV) mit $Z = CH_3CO$- zuerst alkalisch hydrolysiert unter Bildung eines Zwischenprodukts der Formel (IV) mit Z = H, danach wird es in einem mineralsauren Medium intramolekular zum Thiophenderivat der Formel (I) cyclisiert. Auch das von Isaka et al (vgl. Yakugaku Zasshi, 88 (4) (1968), Seiten 422-427) hergestellte Isothiuroniumsalz wird zunächst alkalisch hydrolysiert und dann in einem mineralsauren Medium intramolekular zum Thiophenderivat der Formel (I) cyclisiert. Zwischenprodukte der Formel (IV) können in einem sauren Medium in einem Verfahrensschritt durch Hydrolyse und intramolekulare Cyclisierung in Thiophenderivate der Formel (I) überführt werden (vgl. Zavyalov et al in "Izvestia Akademia Nauk. SSSR Ser. Chim." (1980), Seiten 1 943 bis 1 945, UDSSR-PS 579 767 und PCT-Anmeldung WO 86/02069).

Bei Nacharbeitung der obengenannten Verfahren zur Herstellung von Thiophenderivaten der Formel (I) hat sich jedoch gezeigt, daß diese Verfahren für die g roßtechnische Herstellung, insbesonders für die industrielle Durchführung, ungeeignet sind, da sie eine Reihe von gravierenden Nachteilen aufweisen:

(1) Das als Ausgangsprodukt verwendete, 1,3-Diacyl-4-halogenmethyl-5-(5-alkoxy-carbonylpentanoyl)-4-imidazolon-2 der Formel (II) wird hergestellt durch Halogenierung einer Verbindung der Formel (V)

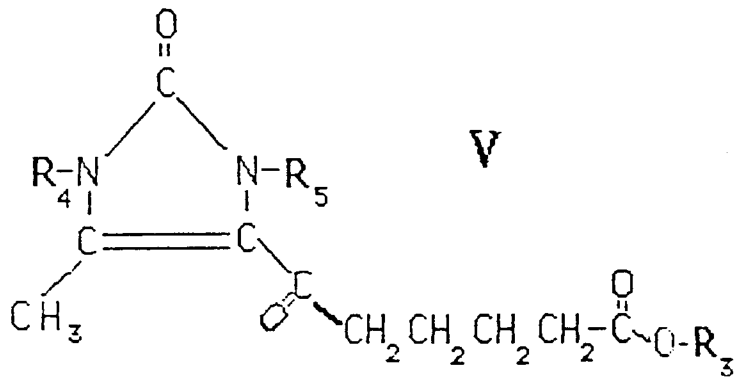

V

worin $R_3$, $R_4$ und $R_5$ die oben angegebenen Bedeutungen haben (vgl. Duschinsky und Dolan in "Am. Soc." 70 (1948), Seiten 657 bis 662).

Das dabei erhaltene rohe Halogenierungsprodukt der Formel (II) muß Umkristallisation auf einen hohen Reinheitsgrad gebracht werden, ehe es zur Umsetzung nach bekannten Verfahren eingesetzt werden kann. Geschieht diese Vorreinigung nicht, entstehen im Laufe der nachfolgenden chemischen Umsetzung äußerst schwer abtrennbare Nebenprodukte, die bei der katalytischen Hydrierung der gewonnenen Thiophenderivate der Formel (I) zur vorzeitigen Vergiftung der Edelmetallkatalysatoren und zur Ausbeuteminderung an (±)-Biotin führen;

(2) Die nach bekannten Verfahren hergestellten S-haltigen Zwischenprodukte der Formel (IV)stellen labile und zer-setzliche Verbindungen dar, die nicht nur in technischem Maßstab schwierig zu handhaben sind, sondern auch noch der zusätzlichen Reinigung durch Umkristallisation bedürfen. Nach den bekannten Verfahren gewinnt man die rohen Zwischenprodukte der Formel (IV) durch Eindampfen des Reaktionsansatzes in Form von Konzentraten. Die Verunreinigungen in solchen Konzentraten stellen in der Regel Spaltprodukte der rohen Zwischenprodukte oder verhältnismäßig niedermolekulare Substanzen dar. Es hat sich als sehr schwierig und verlustreich erwiesen, diese Fremdstoffe zur Isolierung der reinen Zwischenprodukte der Formel (IV)aus den Konzentraten zu entfernen;

(3) Um in großtechnischen Maßstab zu reinen Produkten zu gelangen, müssen bei den bekannten Verfahren zur Herstellung von Thiophenderivaten der Formel (I) äußerst langsame zeitraubende Kristallisationsverfahren angewendet werden. Häufig tritt der Fall ein, daß beim zu schnellen Kristallisieren der erhaltenen gereinigten Lösungen kein kristalliner Niederschlag, sondern zuerst eine Trübung und dann eine ölige Ausscheidung entstehen, die einer weiteren Reinigung bedarf, was die Ausbeute an kristallinem Endprodukt erheblich vermindert;

(4) Die bekannten Verfahren haben außerdem den Nachteil, daß einige zu niedrigen Ausbeuten von höchstens 72% führen, andere Schwierigkeiten bei der großtechnischen Anwendung mit sich bringen, die Verwendung teurer Lösungsmitteln erfordern oder die direkte Kristallisation nicht gestatten.

Aufgabe der Erfindung war es daher, auf technisch einfache und wirtschaftliche Weise, ausgehend von einem rohen Halogenierungsprodukt der nachstehend angegebenen allgemeinen Formel (II), Thiophenderivate der nachstehend angegebenen allgemeinen Formel (I) herzustellen, die eine für die spätere Hydrierung ausreichende Reinheit haben.

Es wurde nun gefunden, daß diese Aufgabe erfindungsgemäß gelöst werden kann, wenn man die Synthese der Thiophenderivate der Formel (I) wie nachstehend beschrieben durchführt.

Gegenstand der Erfindung ist ein Verfahren zur Herstellung von Thiophenderivaten der allgemeinen Formel

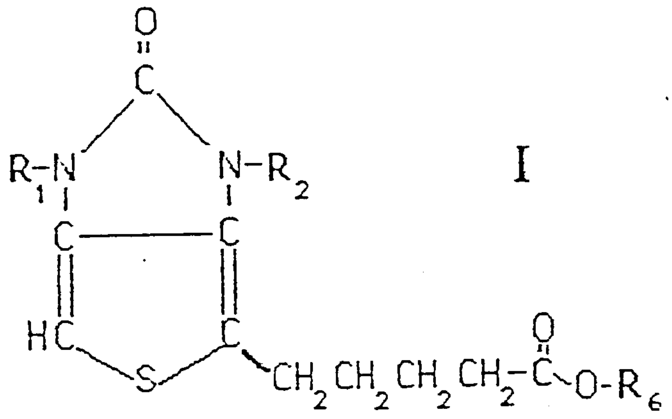

I

worin $R_1$ und $R_2$, die gleich oder verschieden sind, jeweils Wasserstoff oder niederes Acyl und $R_6$ Wasserstoff oder niederes Alkyl bedeuten,

das dadurch gekennzeichnet ist, daß man

(a) eine Verbindung der allgemeinen Formel

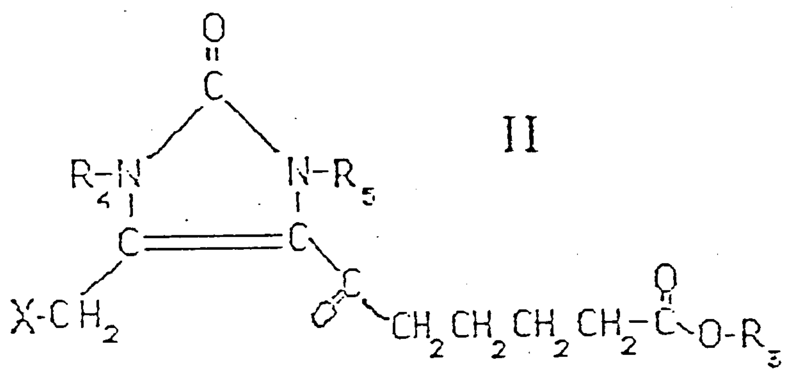

II

worin $R_2$ niederes Alkyl, $R_4$ und $R_5$ niederes Acyl und X Chlor, Brom oder Jod bedeuten, mit einem stark basischen quaternären Ammonium-Anionenaustauscherharz in der Monothiophosphatform bei einer Temperatur von -10 bis +30 °C über eine Reaktionszeit von 10 bis 200 Stunden umsetzt unter Bildung einer polymeren Verbindung der allgemeinen Formel

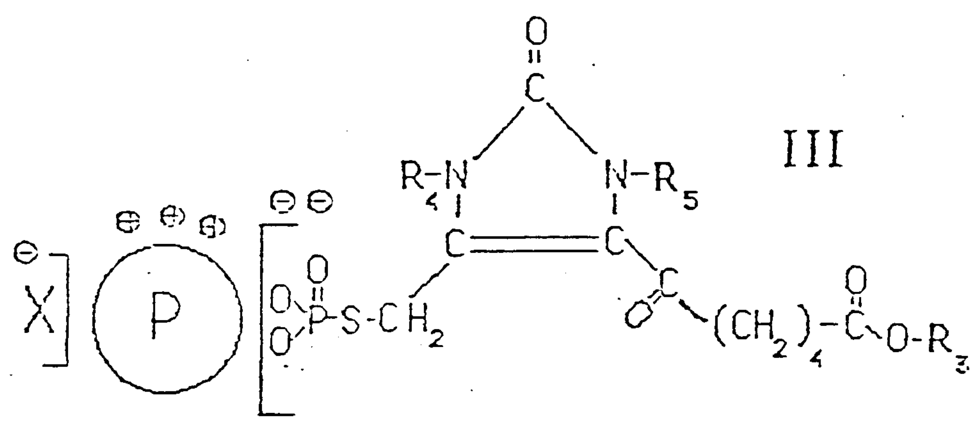

III

worin $R_3$, $R_4$ und $R_5$ die oben angegebenen Bedeutungen haben und $(P)^{+++}$ ein Polymerkation darstellt,

(b) das in der Stufe (a) erhaltene polymere Zwischenprodukt der Formel (III) von nicht-umgesetzten Begleitstoffen abtrennt durch Auswaschen mit einer die Begleitstoffe lösenden Waschflüssigkeit und dann

(c) das ausgewaschene polymere Zwischenprodukt der Formel (III) in saurem Medium bei Temperaturen von +10 bis 60°C über 1 bis 20 Stunden zum Thiophenderivat der Formel (I) umsetzt.

Nach dem erfindungsgemäßen Verfahren lassen sich Thiophenderivate der Formel (I) in hoher Reinheit auf technisch einfache und wirtschaftliche Weise aus dem rohen Halogenierungsprodukt der Formel (II) herstellen. Das dabei in hohen Ausbeuten erhaltene Endprodukt läßt sich ohne weitere Vorreinigung zu dem Vitamin (∓)-Biotin hydrieren.

Gegenstand der Erfindung sind ferner die bei der Durchführung des erfindungsgemäßen Verfahrens als Zwischenprodukte auftretenden neuen polymeren Verbindungen der vorstehend angegebenen allgemeinen Formel

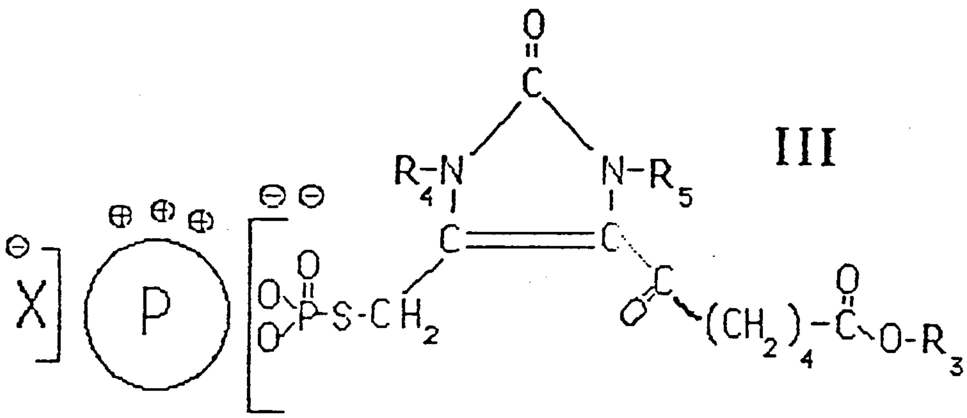

III

worin $R_3$ niederes Alkyl und $R_4$ und $R_5$ niederes Acyl bedeuten.

Das erfindungsgemäße Verfahren zur Herstellung der Thiophenderivate der Formel (I) und der als Zwischenprodukte auftretenden neuen polymeren Verbindungen der Formel (III) wird nachstehend näher erläutert.

Bei der Durchführung des erfindungsgemäßen Verfahrens wird in der Stufe (a) zunächst ein neues polymeres Zwischenprodukt der Formel (III) hergestellt durch Umsetzung einer Halogenverbindung der Formel (II) mit einem stark basischen quaternären Ammonium-Anionenaustauscherharz in der Monothiophosphatform. Geeignete stark basische quaternäre Ammonium-Anionenaustauscherharze sind dem Fachmann bekannt (vgl. K. Dorfner, "Ionenaustauscher", 3. Ausgabe, De Gruyter, Berlin 1970).

Zur Herstellung des erfindungsgemäßen polymeren Zwischenproduktes der allgemeinen Formel (III) können sehr verschiedenartige stark basische Anionenaustauscherharze eingesetzt werden, wobei solche bevorzugt sind, die eine makroporöse Struktur aufweisen. Besonders bevorzugt sind solche Anionenaustauscherharze, die eine makroporöse Struktur haben und ein mit Divinylbenzol vernetztes Polystyrolgerüst enthalten oder aus einem solchen Gerüst bestehen. Die quaternären Ammoniumgruppen können über einen beliebigen divalenten Rest, z.B. Alkylen mit vorzugsweise 1 bis 4 Kohlenstoffatomen wie Methylen, an den Träger gebunden sein. Das Stickstoffatom der quaternären Ammoniumgruppen kann gleiche oder verschiedene Substituenten tragen, wobei ggf. (z.B. durch Hydroxyl) substituierte Alkylreste mit 1 bis 4 Kohlenstoffatomen, insbesondere Methyl oder Ethyl, aufgeführt seien. Beispielhaft seien hierzu

$-CH_2-N(CH_3)_3$ und 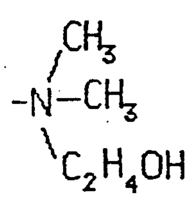

genannt. Die Anionenaustauscher kommen üblicherweise mit den Gegenionen Chlorid oder Acetat in den Handel und können durch Behandlung mit einer wäßrigen Lösung von Alkalioder Erdalkalisalzen der Monothiophosphorsäure in die Monothiophosphatform überführt werden.

Die Methoden und Bedingungen, unter denen der Anionenaustausch stattfindet, sind dem Fachmann bekannt. Bei der bevorzugten Verfahrensweise wird der Anionenaustauscher in der Chlorid- oder Acetatform in eine Ionenaustauschersäule gefüllt und mit Wasser gewaschen. Anschließend wird von oben oder von

unten in die Säule eine wäßrige LÖsung von Alkali- oder Erdalkalisalzen der Monothiophosphorsäure so lange hindurchgeleitet, bis die Gegenionen im Harz vollständig ausgetauscht sind und sich im Ablauf der Flüssigkeit keine Chlorid- bzw. Acetat-Ionen mehr nach-weisen lassen. Die Harzsäule wird anschließend mit Wasser gewaschen, bis der Ablauf der Flüssigkeit salzfrei ist und dann wird das Waser im Harzbett durch ein geeignetes Lösungsmittel verdrängt.

Geeignete Lösungsmittel zur Umsetzung der rohen Halogenierungsprodukte der Formel (II) mit dem quaternären Ammonium-Anionenaustauscherharz in der Monothiophosphatform sind niedere Alkanole oder Mischungen von niederen Alkanolen mit inerten aprotischen Lösungsmitteln. Derartige geeignete organische Lösungsmittel können auch geringe Mengen Wasser enthalten, ohne das die chemische Reaktion der Umsetzung gestört wird. Mit besonderem Vorteil wird die Umsetzung in Isopropanol als Lösungsmittel vorgenommen. Bei einer weiteren vorteilhaften Arbeitsweise erfolgt die Umsetzung in der Mischung aus einem niederen Alkanol und Dimethylformamid als Lösungsmittel. Die Umsetzung zwischen dem gelösten rohen Halogenierungsprodukt der Formel (II) und dem quaternären Ammonium-Anionenaustauscherharz in der Monothiophosphatform erfolgt bei Temperaturen von -10 bis +30 °C. Die Reaktionsdauer beträgt 10 bis 200, vorzugsweise 30 bis 90 Stunden. Die Reaktion ist beendet, wenn sich 95 bis 99 Mol% des in Lösung vorhandenen Halogenierungsprodukt der Formel (II) mit dem Anionenaustauscherharz zu dem neuen polymeren Zwischenprodukt der Formel (III) umgesetzt haben.

Der Fortgang der Reaktion kann durch Dünnschichtchromatographie (DC) kleiner Proben verfolgt werden. Die DC erfolgte im allgemeinen an Kieselgel mit reinem Essigsäureethylester als Laufmittel. Der Vorteil dieses erfindungsgemäßen Reaktionsschrittes besteht darin, daß aus dem rohen Halogenierungsprodukt der Formel (II) ein stabiles polymeres Zwischenprodukt der Formel (III) erzeugt wird und auf mindestens zwei Umkristallisationen und zwei Eindampfungsvorgänge verzichtet werden kann.

Das in der Stufe (a) gewonnene neue polymere Zwischenprodukt der Formel (III) weist die physikalische Beschaffenheit des eingesetzten Anionenaustauscherharzes auf und ist daher sehr leicht zu handhaben. Dieser erfindungsgemäße Vorteil wird im nächsten erfindungsgemäßen Verfahrensschritt (b) ausgenutzt, indem das neue polymere Zwischenprodukt der Formel (III) mit Lösungsmitteln ausgewaschen wird, wodurch fremde Begleitstoffe und nichtumgesetztes Halogenierungsprodukt der allgemeinen Formel (II) ausgeschwemmt werden.

Vorteilhaft werden für diesen Reinigungsschritt polare Lösungsmittel eingesetzt. Als solche sind zu nennen: Niedere Alkanole, aprotische Lösungsmittel wie Acetonitril, Dimethylformamid, Dimethylsulfoxid, Tetramethylensulfon, Dioxan, Tetrahydrofuran und niedere Ketone. Weitere geeignete Lösungsmittel, um die Verunreinigungen aus dem neuen Zwischenprodukt der allgemeinen Formel (III) auszuwaschen, sind niedere aliphatische Carbonsäuren wie Essigsäure und Propionsäure. Der Verfahrensschritt der Auswaschung stellt keine technischen Ansprüche und erfordert keine speziellen Apparaturen, da das Zwischenprodukt der Formel (III) in leicht zu handhabenden kleinen Plastikkugeln vorliegt. Der Fortgang der Auswaschung kann nach bekannten Methoden überwacht werden. Eine sichere Methode ist die Entnahme von Proben aus der Waschlösung. Diese Proben werden schonend eingedampft. Der zurückbleibende Verdampfungsrückstand ist ein Maß für den Grad der Auswaschung.

Das durch Auswaschung in der Stufe (b) gereinigte neue polymere Zwischenprodukt der Formel (III) kann nun in an sich bekannter Weise in einem sauren Medium durch Hydrolyse und Cyclisierung in Thiophenderivate der allgemeinen Formel (I) überführt werden.

Bei der Hydrolyse wird die P-S-Bindung im polymeren Zwischenprodukt der Formel (III) aufgespalten, indem das Mercaptan der allgemeinen Formel (IV)mit Z = H entsteht.

Als Säuren für dieses Reaktionsmedium kommen beispielsweise in Betracht: verdünnte Mineralsäuren und organische starke Säuren wie Sulfonsäuren. Insbensondere hat es sich als vorteilhaft zur Durchführung des erfindungsgemäßen Verfahrens erwiesen, solche Säuren zur Erzeugung des sauren Mediums zu verwenden, deren Säuredissoziationsexponent pKs, in Wasser gemessen, $< 3$ ist. Um Lösungsmittel zu sparen, kann man in vorteilhafter Weise für die Auswaschung nach Verfahrensschritt (b) und für die Hydrolyse/Cyclisierung nach Verfahrensschritt (c) das gleiche Medium verwenden. Beispielsweise kann man die Auswaschung mit Essigsäure vornehmen und dann anschließend die Hydrolyse/Cyclisierung mit HCl/Essigsäure durchführen. Es hat sich als notwendig erwiesen, zur Erzeugung des sauren Mediums mindestens 2 Äquivalente Säure pro g-Atom des im polymeren Zwischenprodukt gebundenen Phospors einzusetzen. Das saure Medium dieses Verfahrensschrittes kann z.B. eine verdünnte wäßrige Mineralsäure sein. In diesem Fall wird das gebildete Thiophenderivat der allgemeinen Formel (I) infolge seiner Unlöslichkeit in Wasser an dem Anionenaustauscherharz adsorbiert bleiben und kann dann nach dem Auswaschen mit Wasser mittels verdünnter Alkalilauge aus dem Harz eluiert werden. Dient Essigsäure oder ein niederer Alkanol als Lösungsmittel für die Mineralsäure oder die starke organische Säure, so bleibt die gebildete Thiophenverbindung der allgemeinen Formel (I) in gelöster Form vorhanden und kann durch Auswaschen

z.B. mit dem gleichen Lösungsmittel vom Anionenaustauscherharz abgetrennt werden.

Die Umsetzung zwischen dem gereinigtem polymeren Zwischenprodukt der Formel (III) und dem sauren Medium erfolgt bei Temperaturen von +10 °C bis +60 °C. Die Reaktionsdauer beträgt 1 bis 20, vorzugsweise 3 bis 8 Stunden. Die Reaktion ist beendet, wenn sich 95 bis 99 Mol% des neuen polymeren Zwischenprodukt der Formel (III) chemisch umgesetzt haben.

Wie aus der vorstehenden Verfahrensbeschreibung ersichtlich, weist das erfindungsgemäße Verfahren wesentliche Vorteile gegenüber den bekannten Verfahren zur Herstellung der Thiophenderivate der allgemeinen Formel (I) auf. Insbesondere unterliegt das erfindungsgemäße Verfahren keiner der bislang vorhandenen technischen und apparativen Beschränkungen. Es macht die Anwendung großer Mengen Lösungsmittel überflüssig, erfordert bis zum gewünschten Endprodukt der Formel (I) nur eine Kristallisation, wo zuvor vier und mehr erforderlich waren. Das Eindampfen der verwendeten Lösungsmittel zwecks Rückgewinnung erfolgt ohne Kontakt mit dem Zwischenprodukt der Formel (III) und kann daher unter sehr wirtschaflichen Bedingungen durchgeführt werden. Demgemäß eröffnet das erfindungsgemäße Verfahren eine neue vorteilhafte Synthese von Thiophenderovatem der Formel (I) und führt über neue polymere Zwischenprodukte der Formel (III).

Die Erfindung wird durch die folgenden Beispiele näher erläutert.

Beispiel 1

A1) Herstellung von 1,3-Diacetyl-4-bromomethyl-5-(5-ethoxycarbonylpentanoyl)-4-imidazolon-2

Die als Ausgangsprodukt benötigte Verbindung der Formel (V)

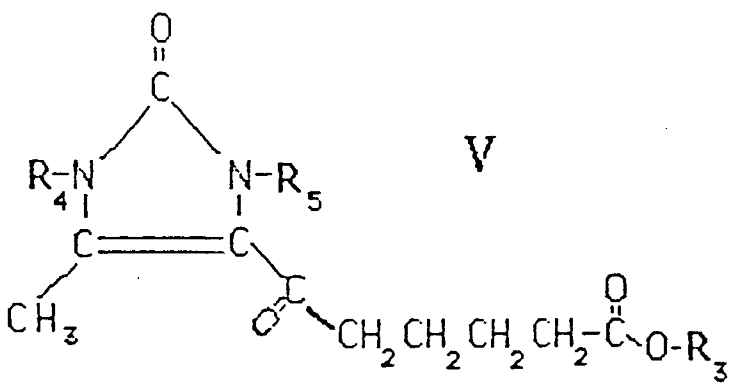

V

mit $R_4$ = $R_5$ = $CH_3CO$- und $R_3$ = $C_2H_5$- [1,3-Diacetyl-4-methyl-5-(5-ethoxycarbonylpentanoyl)-4-imidazolon-2 ($C_{16}H_{22}O_6N_2$) MG 338,36] wurde nach der Vorschrift von R. Duschinsky und L.A. Dolan, "Am.Soc" 67(1945), S. 2079-2084, hergestellt:

100 g (0,295 Mol) 1,3-Diacetyl-4-methyl-5-)5-ethoxycarbonylpentanoyl)-4-imidazolon-2 wurden unter Rühren in 1 l über $P_4O_{10}$ absolutiertem $CCl_4$ gelöst und anschließend wurden 52,5 g (0,295 Mol) N-Bromsuccinimid und 3 g Succinimid zugegeben. Der Ansatz wurde erwärmt unb bei beginnendem Sieden wurden je 100 mg Benzoylperoxid und Azoisobutyronitril eingebracht. Nun wurde das Anspringen der Reaktion - erkenntlich am Aufsteigen des gebildeten Succinimids zur Flüssigkeitsoberfläche - abgewartet. Nach 2 Stunden wurde noch die gleiche Menge Benzolyperoxid und Azoisobutyronitril zugesetzt. Nach 4 Stunden Reaktionszeit war die Bromierung beendet. Der Ansatz wurde abgekühlt und das gebildete Succinimid abfiltriert und mit 200 ml $CCl_4$ gewaschen. Das Filtrat wurde bei 20 °C im Wasserstrahlvakuum bis zu einem öligen Sirup eingedampft. Das rohe Halogenisierungsprodukt wog 170 g und enthielt 113 g = 0,271 Mol (92 Mol% Ausbeute) 1,3-Diacetyl-4-bromomethyl-5-(5-ethoxy-carbonylpentanoyl)-4-imidazolon-2 ($C_{16}H_{21}BrO_6N_2$) (MG 417,26), 7 g Succinimid, 10 g unbekannte Substanzen, der Rest zu 170 g war $CCl_4$.

A2) Das zum Einsatz kommende stark basische makroporöse Anionenaustauscherharz hat die Handelsbezeichnung "Amberlyst A26". (Amberlyst® ist ein Warenzeichen der Rohm & Haas Co.). Es enthält die funktionelle Gruppe $-CH_2-\overset{\oplus}{N}(CH_3)_3$ und liegt in der Chloridform vor.

In einer Ionenaustauschersäule mit dem Innendurchmesser 4 cm wurde durch Einschlämmen mit Wasser ein 25 cm hohes Harzbett mit "Amberlyst A 26" erzeugt. Durch dieses Harzbett wurden von oben nach unten 80 g Natrium-monothiophosphat $Na_3PO_3S.12H_2O$ (0,202 Mol), gelöst in 2 l Wasser, hindurchgeleitet.

Die Harzsäule wurde mit Wasser salzfrei gewaschen und das Wasser mit Dioxan verdrängt. Eine Elementaranalyse des Ionenaustauscher ergab für das Verhältnis (g-Atom zu g-Atom) P : S : Cl die Werte 1:0,93:0,04. Die so hergestellte Anionenaustauschermasse "A" zeigte während einer Beobachtungsdauer von 3 Wochen bei Raumtemperatur keine Zersetzungserscheinungen.

50 g des oben hergestellten rohen 1,3-Diacetyl-4-bromomethyl-5-(5-ethoxy-carbonylpentanoyl)-4-imidazolon-2 (entsprechend 0,079 g-Atom Brom) wurden in einer Mischung aus 500 ml Dioxan und 50 ml Dimethylformamid gelöst. Die Lösung wurde 96 Stunden lang bei 20 °C mit 320 ml der Anionenaustauschermasse "A" (0,08 g-Atom Schwefel enthaltend) geschüttelt.

B) Danach wurde der Ansatz auf eine Filternutsche gegeben und das gewonnene neue polymere Zwischenprodukt zur Entfernung der Fremdstoffe 2 mal mit je 400 ml Dioxan gewaschen. Anschließend wurde die Harzmasse noch 2 mal mit 600 ml Methanol gewaschen.

Eine Elementaranalyse des neuen polymeren Zwischenprodukts ergab die Verhältnisse (g-Atom zu g-Atom) S : Br von 1:0,94. Es hatten sich dementsprechend 94 Mol% des eingesetzten 1,3-Diacetyl-4-bromomethyl-5-(5-ethoxy-carbonylpentanoyl)-4-imidazolon-2 mit dem Monothiophosphat umgesetzt. Das neue polymere Zwischenprodukt wies die Morphologie des eingesetzten Anionenaustauscherharzes auf und hatte die Formel

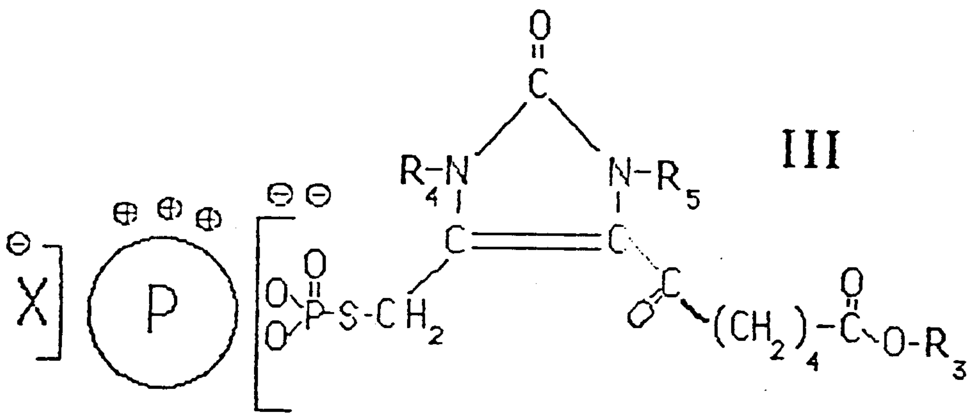

III

mit X = Br, $R_4$ und $R_5$ = $CH_3CO$- und $R_3 = C_2H_5$.

C) Das ausgewaschene methanolfeuchte polymere Zwischenprodukt aus dem vorhergehenden Verfahrensschritt wurde anschließend mit einer Lösung von 21 g Salzsäure 31% (0,175 Mol) und 19 g Toluolsulfonsäure-monohydrat (0,1 Mol) in 500 ml Methanol 8 Stunden bei 58 °C bis 60 °C geschüttelt. Danach wurde die Lösung des gebildeten Thiophenderivates in Methanol von dem jetzt schwefelfreien Anionenaustauscherharz auf einer Druckfilternutsche abgetrennt. Das Harzbett wurde mit 1 l Methanol nachgewaschen und die vereinigten Filtrate (ca. 1,4 l) im Vakuum eingedampft. Der eingedampfte Rückstand wurde mit 200 ml 0,1 N Kaliumphosphatlösung auf pH 6 eingestellt und ergab 16,3 g (0,064 Mol) 2,3,8,9-Tetrahydrobiotin-methylester der Formel

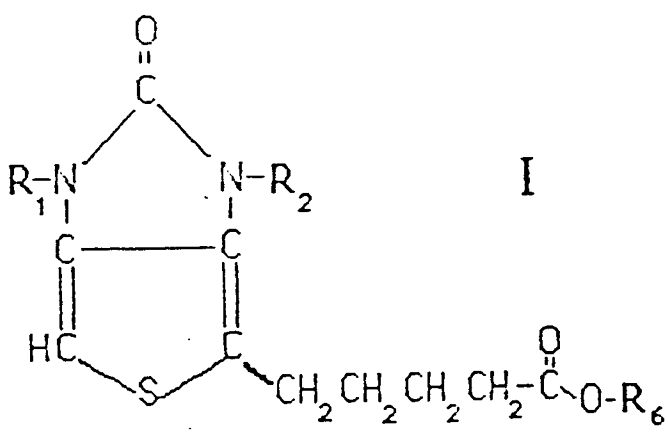

I

mit $R_1$ = $R_2$ = H und $R_6$ - $CH_3$-; $C_{11}H_{14}N_2O_3S$; MG 254,30, F. 142 °C (80 Mol% Ausbeute, bezogen auf

das eingesetzte rohe Bromierungsprodukt).

Das verwendete Anionenaustauscherharz wurde durch Auswaschen mit 1 N Natronlauge, Spülen mit Wasser und Durchleiten von 0,1 N Salzsäure regeneriert.

Beispiel 2

A) Das als Anionenaustauscherharz verwendete Material hatte die Handelsbezeichnung "DOWEX MSA-1". (DOWEX® ist ein Warenzeichen der DOW CHEMICAL Co.). Das Harz liegt in Form opaker Kugeln mit einem durchschnittlichen Kugeldurchmesser von 0,3 bis 1,2 mm vor. Es ist ein stark basischer Anionenaustauscher mit makroporöser Struktur. Die funktionelle Gruppe war Methylen-trimethylammonium, das Gegenion im Lieferzustand war Chlorid.

300 ml dieses wasserfeuchten Harzes wurden in die Ionenaustauschersäule des Beispiels 1 gegeben. Durch Auswaschen mit 1 l 1 N Natriumacetatlösung wurde das Harz in die Acetatform überführt. Nach dem Spülen mit Wasser wurde durch dieses Harzbett von oben nach unten eine Lösung von 80 g Natrium-monothiophosphat $Na_3PO_3S.12H_2O$ (0,202 Mol) in 2 l Wasser hindurchgeleitet.

Die Harzsäule wurde mit Wasser salzfrei gewaschen und das Wasser mit Isopropylalkohol verdrängt. Eine Elementaranalyse des Ionenaustauschers ergab für das Verhältnis (g-Atom zu g-Atom) P:S:Cl die Werte 1:0,98:0,01. Die gesamte alkoholfeuchte Harzsäule enthielt 0,08 g-Atom bebundenen Schwefel als Monothiophosphat und wurde vollständig in ein Schüttelgefäß überführt.

50 g des in Beispiel 1 hergestellten rohen Bromierungsprodukts 1,3-Diacetyl-4-bromomethyl-5-(5-ethoxy-carbonylpentanoyl)-4-imidazolon-2 (entsprechend 0.079 g-Atom Brom) wurden in einer Mischung aus je 300 ml Isopropylalkohol und Acetonitril gelöst und in dem Schüttelgefäß mit dem Anionenaustauscherharz in der Monothiophosphatform 60 Stunden bei 30 ° C geschüttelt.

B) Danach wurde der Ansatz auf eine Filternutsche gegeben und das gewonnene neue polymere Zwischenprodukt zur Entfernung der Femdstoffe 3 mal mit je 400 ml Essigsäure gewaschen. Die ausgewaschene Harzmasse stellte das neue polymere Zwischenprodukt dar. Eine Elementaranalyse des Harzes ergab ein Verhältnis (g-Atom zu g-Atom) S:Br von 1:0,96. Es hatten sich dementsprechend 96 Mol% des eingesetzten 1,3-Diacetyl-4-bromomethyl-5-(5-ethoxy-carbonylpentanoyl)-4-imidazolon-2 mit dem Monothiophosphat umgesetzt.

C) Das in Essigsäure suspendierte neue polymere Zwischenprodukt wurde in das Schüttelgefäß zurückgegeben und mit einer Lösung von 15 g Salzsäure wasserfrei (0,4 Mol) in 500 ml Eisessig 8 Stunden bei 28 ° C geschüttelt.

Danach wurde die Lösung des gebildeten Thiophenderivats in Essigsäure von dem jetzt schwefelfreien Anionenaustauscherharz auf einer Druckfilternutsche abgetrennt. Das Harzbett wurde mit 1 l Eisessig nachgewaschen und die vereinigten Filtrate (ca. 1,6 l) nach Zugabe von 32 g wasserfreiem Natriumacetat im Vakuum bei 25 ° C eingedampft. Der eingedampfte Rückstand wurde mit 200 ml Eiswasser verrührt, mit Salzsäure auf pH 2 eingestellt, der entstehende Niederschlag abfiltriert und aus Methanol umkristallisiert. Es wurden 14,4 g (0,060 Mol) 2,3,8,9-Tetrahydrobiotin der Formel I erhalten:

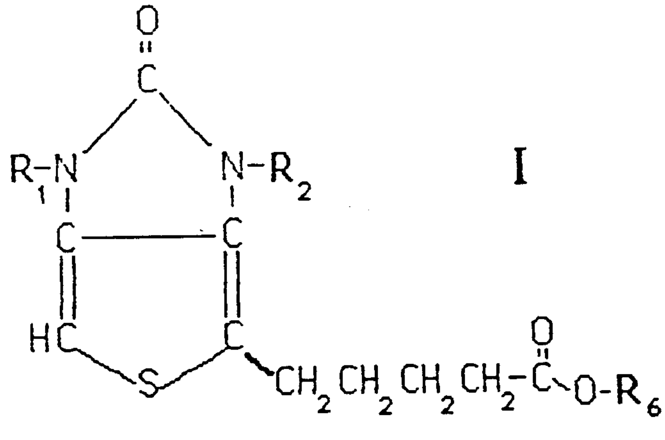

I

mit $R_1 = R_2 = R_6 = H$; $C_{10}H_{12}N_2O_3S$; MG 240,28 F. 240 ° C (entsprechend 75 Mol% Ausbeute, bezogen auf das eingesetzte rohe Bromierungsprodukt).

**Patentansprüche**

**1.** Verfahren zur Herstellung von Thiophenderivaten der allgemeinen Formel

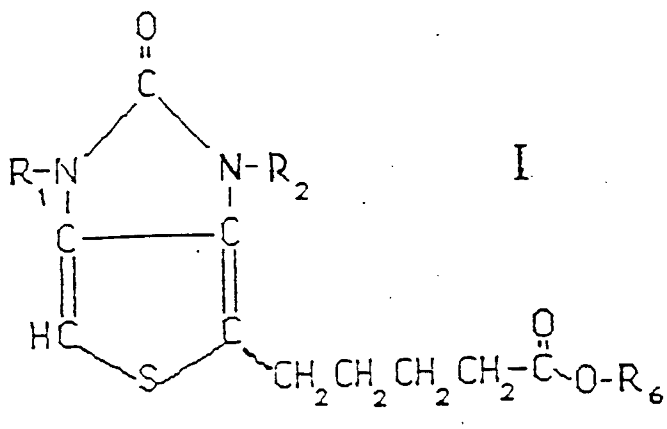

I

worin $R_1$ und $R_2$, die gleich oder verschieden sind, jeweils Wasserstoff oder niederes Acyl und $R_6$ Wasserstoff oder niederes Alkyl bedeuten,
dadurch **gekennzeichnet,** daß man
(a) eine Verbindung der allgemeinen Formel

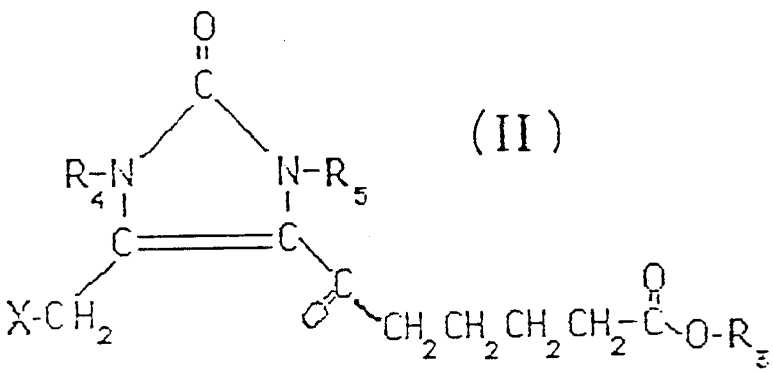

(II)

worin $R_3$ niederes Alkyl, $R_4$ und $R_5$ niederes Acyl und X Chlor, Brom oder Jod bedeuten,
mit einem stark basischen quaternären Ammonium-Anionenaustauscherharz in der Monothiophosphatform bei einer Temperatur von -10 bis +30°C über eine Reaktionszeit von 10 bis 200 Stunden umsetzt unter Bildung einer polymeren Verbindung der allgemeinen Formel

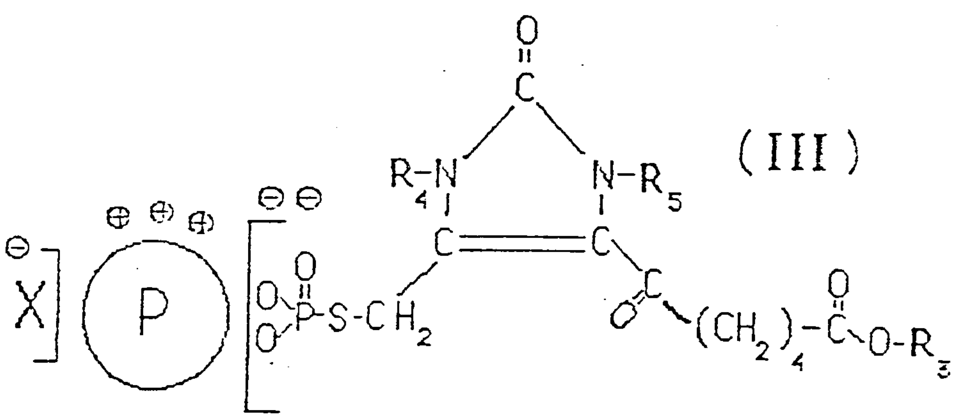

(III)

worin $R_3$, $R_4$ und $R_5$ die oben angegebenen Bedeutungen haben und $(P)^{+++}$ ein Polymerkation

darstellt,
(b) das in der Stufe (a) erhaltene polymere Zwischenprodukt der Formel (III) von nicht-umgesetzten Begleitstoffen abtrennt durch Auswaschen mit einer die Begleitstoffe lösenden Waschflüssigkeit und dann
(c) das ausgewaschene polymere Zwischenprodukt der Formel (III) in saurem Medium bei Temperaturen von +10 bis +60 ° C für 1 bis 20 Stunden zum Thiophenderivat der Formel (I) umsetzt.

2. Verfahren nach Anspruch 1, dadurch gekennzeichnet, daß in der Stufe (a) ein Anionenaustauscherharz mit einer makroporösen Struktur und einem mit Divinylbenzol vernetzten Polystyrolgerüst verwendet wird, in dem die quaternären Ammoniumgruppen über einen divalenten Rest an den Träger gebunden sind.

3. Verbindung der allgemeinen Formel

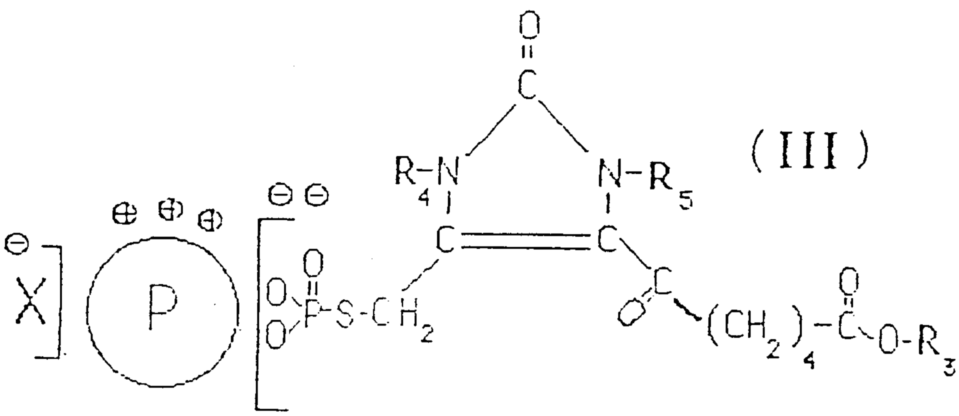

(III)

worin $R_3$ niederes Alkyl, $R_4$ und $R_5$ niederes Acyl und $(P)^{+++}$ ein Polymerkation bedeuten.

**Claims**

1. A method of producing thiophene derivatives having the general formula

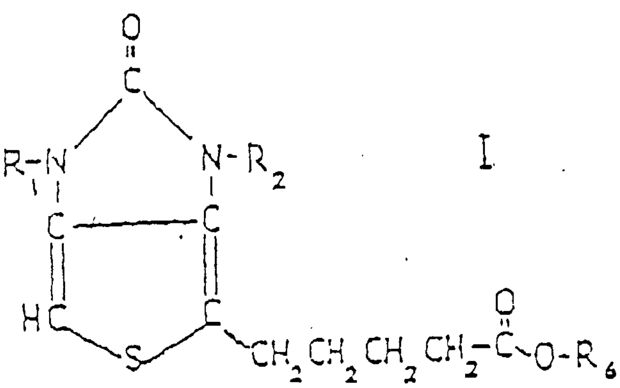

I.

in which $R_1$ and $R_2$, which are the same or different, denote hydrogen or low acyl and $R_6$ denotes hydrogen or low alkyl, characterised in that
(a) a compound having the general formula

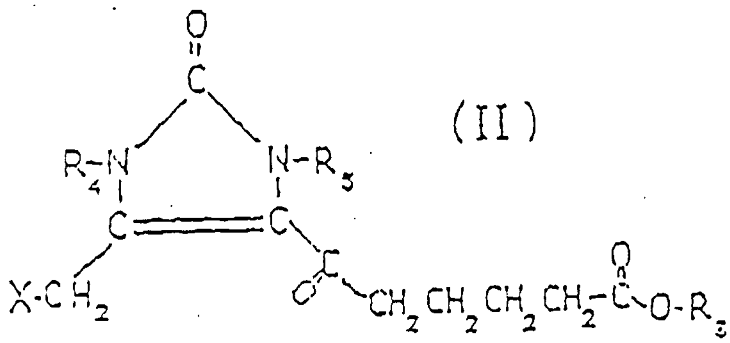

(II)

in which $R_3$ denotes low alkyl, $R_4$ and $R_5$ denote low acyl and X denotes chlorine, bromine or iodine, is reacted with a strongly basic quaternary ammonium anion exchanger resin in the monothiophosphate form at a temperature of -10 to +30°C for 10 to 200 hours, producing a polymeric compound having the general formula

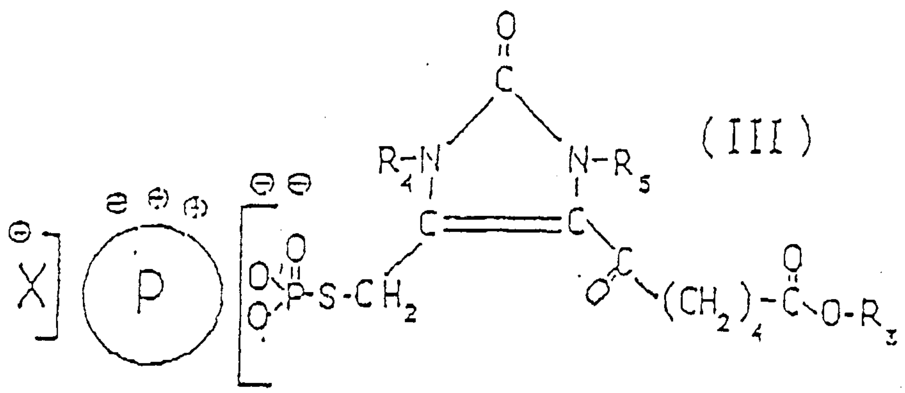

(III)

in which $R_3$, $R_4$ and $R_5$ have the meanings given previously and $(P)^{+++}$ denote, a polymer cation,
(b) the polymeric intermediate product obtained in step (a) and having the formula (III) is separated from unreacted accompanying substances by washing with a washing liquid which dissolve, the accompanying substances, and
(c) the washed polymeric formula (III) intermediate prroduct is reacted in an acid medium at temperatures of +10 to +60°C for 1 to 20 hours to obtain the formula (I) thiophene derivative.

**2.** A method according to claim 1, characterised in that an anion exchanger resin used in step (a) has a macroporous structure and a polystyrene framework cross-linked with divinyl benzene and in which the quaternary ammonium groups are bonded to the carrier by a divalent radical.

**3.** A compound having the general formula

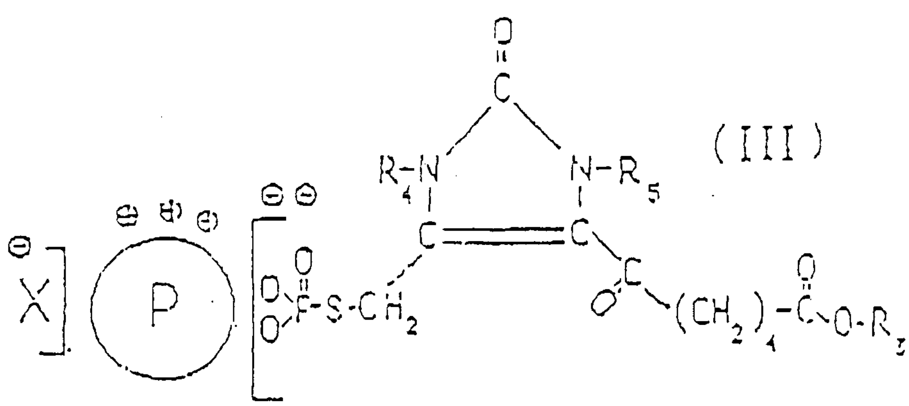

(III)

in which $R_3$ denotes low alkyl, $R_4$ and $R_5$ donote low acyl end $(P)^{+++}$ denotes a polymer cation.

**Revendications**

**1.** Procédé pour la fabrication de dérivés de thiophène de formule générale

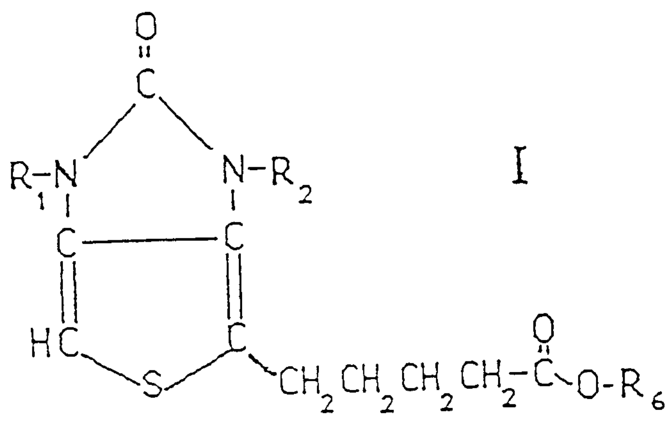

I

dans laquelle $R_1$ et $R_2$, qui sont identiques ou différents, représentent chacun l'hydrogène ou un groupe acyle inférieur et $R_6$ représente l'hydrogène ou un groupe alkyle inférieur,
caractérisé en ce que
(a) on fait réagir un composé de formule générale

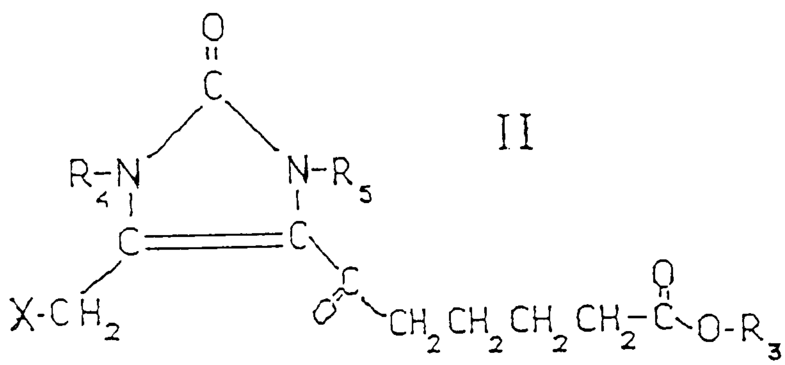

II

dans laquelle $R_3$ représente un groupe alkyle inférieur, $R_4$ et $R_5$ représentent un groupe acyle inférieuret X représente le chlore, le brome ou l'iode, avec une résine échangeuse d'anions du type ammonium quaternaire fortement basique sous la forme monothiophosphate à une température de -10 à +30 °C pendant une durée de réaction de 10 à 200 h avec formation d'un composé polymère de formule générale

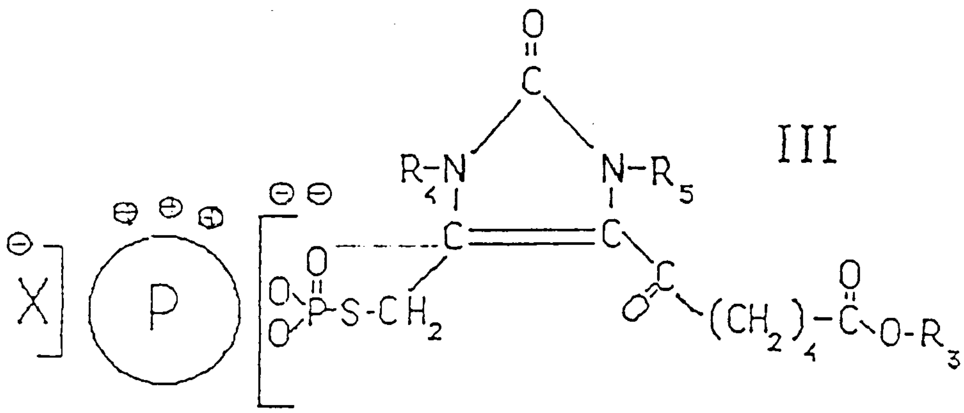

III

dans laquelle $R_3$, $R_4$ et $R_5$ ont les significations indiquées ci-dessus et $(P)^{+++}$ représente un cation polymère,

(b) on sépare le produit intermédiaire polymère de formule (III) obtenu à l'étape (a) des impuretés d'accompagnement n'ayant pas réagi par lavage par un liquide de lavage dissolvant les impuretés d'accompagnement et ensuite

(c) on transforme le produit intermédiaire polymère de formule (III) lavé en le dérivé de thiophène de formule (I) en milieu acide à des températures de +10 à +60 ° C pendant 1 à 20 h.

**2.** Procédé selon la revendication 1, caractérisé en ce que l'on utilise dans l'étape (a) une résine échangeuse d'anions de structure macroporeuse et ayant un squelette de polystyrène réticulé par le divinylbenzène, dans lequel les groupes ammonium quaternaire sont liés au support par un reste divalent.

**3.** Composé de formule générale

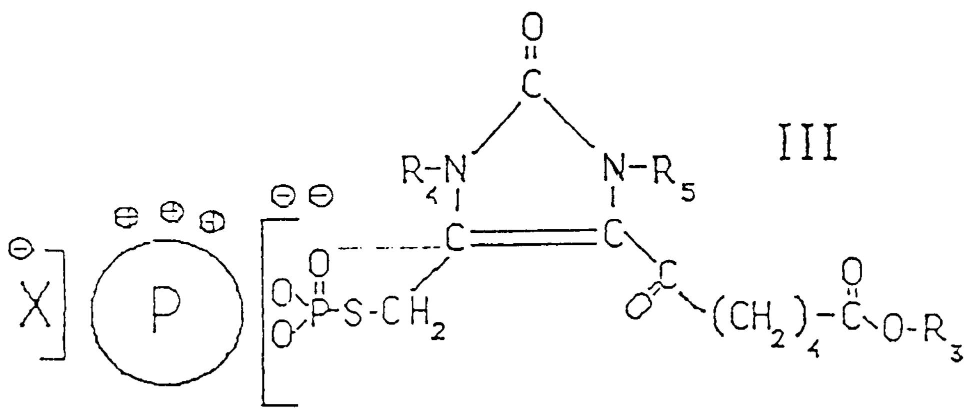

III

dans laquelle $R_3$ représente un groupe alkyle inférieur, $R_4$ et $R_5$ représentent un groupe acyle inférieur et $(P)^{+++}$ représente un cation polymère.